# EUROPÄISCHE PATENTSCHRIFT

(11) **EP 0 438 482 B1**
(45) Veröffentlichungstag und Bekanntmachung des Hinweises auf die Patenterteilung: **10.02.1993**
(21) Anmeldenummer: 89911774.1
(22) Anmeldetag: 11.10.1989
(51) Int. Cl.: A61F 5/44, A61B 5/20, G01N 33/493

(54) **EINRICHTUNG ZUR AUFNAHME UNKONTROLLIERBAR AUSGESCHIEDENEN URINS**
DEVICE FOR COLLECTING UNCONTROLLABLY RELEASED URINE
DISPOSITIF POUR RECUEILLIR L'URINE DE MICTIONS INVOLONTAIRES

(30) Priorität: 12.10.1988 DE 3834725; 22.09.1989 DE 3931659
(43) Veröffentlichungstag der Anmeldung: 31.07.1991
(73) Patentinhaber: RAHE, Martin, D-32609 Hüllhorst (DE)
(72) Erfinder: RAHE, Martin, D-32609 Hüllhorst (DE)
(74) Vertreter: Haft, von Puttkamer, Berngruber, Czybulka
(86) Internationale Anmeldenummer: DE8900648
(87) Internationale Veröffentlichungsnummer: WO9003771

(56) Entgegenhaltungen:
- EP-A- 0 146 691
- DE-A- 3 504 527
- US-A- 3 640 388
- US-A- 3 660 033

## Beschreibung

Die Erfindung betrifft eine Einrichtung zur Aufnahme unkontrollierbar ausgeschiedenen Urins, der durch einen Einleitschlauch in eine durchsichtige Auffangvorrichtung gelangt, wobei zur Kontrolle des aufgefangenen Urins hinsichtlich sich anbahnender und bestehender Keiminfektionen urinexponierte Indikatoren (10) vorgesehen sind.

Eine solche Einrichtung ist aus der DE-A-3 504527 bekannt.

Benutzer der genannten Einrichtung leiden häufig an Infektionskrankheiten, die die ableitenden Harnwege sowie auch Blase und Nieren befallen können. Um diesen Infektionen entgegenzuwirken, ist es bekannt, entzündungshemmende Medikamente einzunehmen. Diese verlieren jedoch nach längerer Einnahme ihre Wirksamkeit. Es sind auch Medikamente bekannt, die ebenfalls oral appliziert werden, die den Urin ansäuern und so die Keimvermehrung verlangsamen. Beide Medikamente haben unerwünschte Nebenwirkungen, die zum Absetzen der Einnahme zwingen. Darüber hinaus ist der Träger gehalten, die Einrichtung täglich zu wechseln und sich im Zweiwochenrhythmus ärztlich untersuchen zu lassen.

Aufgabe der vorliegenden Erfindung ist es, eine Einrichtung der eingangs genannten Art zu schaffen, die es ermöglicht, die Beschaffenheit des Urins zu signalisieren, um rechtzeitig geeignete Maßnahmen zur Vermeidung einer Infektion treffen zu können oder um in deren Anfangsstadium einen Arzt zu konsultieren.

Die Aufgabe wird dadurch gelöst, daß die urinexponierten Indikatoren auf einer in der Auffangvorrichtung befestigten Kontrollkarte aufgetrage sind, und daß die Kontrollkarte auf ihrer urinexponierten Seite mit einer Membran überzogen ist, die einerseits die Urinaufnahme verlangsamt und andererseits einer Ausschwemmung von Indikatorsubstanzen entgegenwirkt.

Jede derzeit gebräuchliche Einrichtung der eingangs genannten Art kann vor Verschweißen des Auffangbeutels innenseitig mit der Kontrollkarte durch Kleben oder Schweißen versehen werden. Die Membran schützt die Indikatoren einerseits aufgrund ihres verlangsamenden Urinzutritts vor Fehlanzeigen. Weder die zu hohe Keimkonzentration des Vorlaufurins noch die typischen Abweichungen im Restharn, sondern die über den gesamten Urininhalt integrierten Werte werden von den Indikatoren angezeigt. Da die Membran auch vor Auswaschen der Indikatoren schützt, können die Anzeigenwerte auch nach vielen Stunden unverfälscht abgelesen werden.

Mit den in den Unteransprüchen enthaltenen Ausgestaltungsmerkmalen ergibt sich insgesamt eine Einrichtung, deren Indikatoren eine genaue Meßwertanzeige ermöglichen.

Nachfolgend ist die Erfindung anhand von in der Zeichnung dargestellten Ausführungsbeispielen naher beschrieben.

Es zeigen:
Fig. 1 eine bekannte Einrichtung zur Aufnahme unkontrollierbar ausgeschiedenen Urins, der durch einen Einleitschlauch in einen durchsichtigen, mit Befestigungsriemen versehenen und leerbaren Auffangbeutel gelangt,
Fig. 2 Einrichtung gem. Fig. 1 mit Kontrollkarte auf der Innenwandung des Auffangbeutels, nach der Erfindung,
Fig. 3 Querschnitt durch die Wandung des Auffangbeutels gem. Fig. 1 mit einer gegenüber Fig. 2 vereinfachten Kontrollkarte,
Fig. 4 Querschnitt wie in Fig. 3, jedoch mit abgewandelter Membrananordnung und
Fig. 5 eine aufgerollte Kontrollkarte.

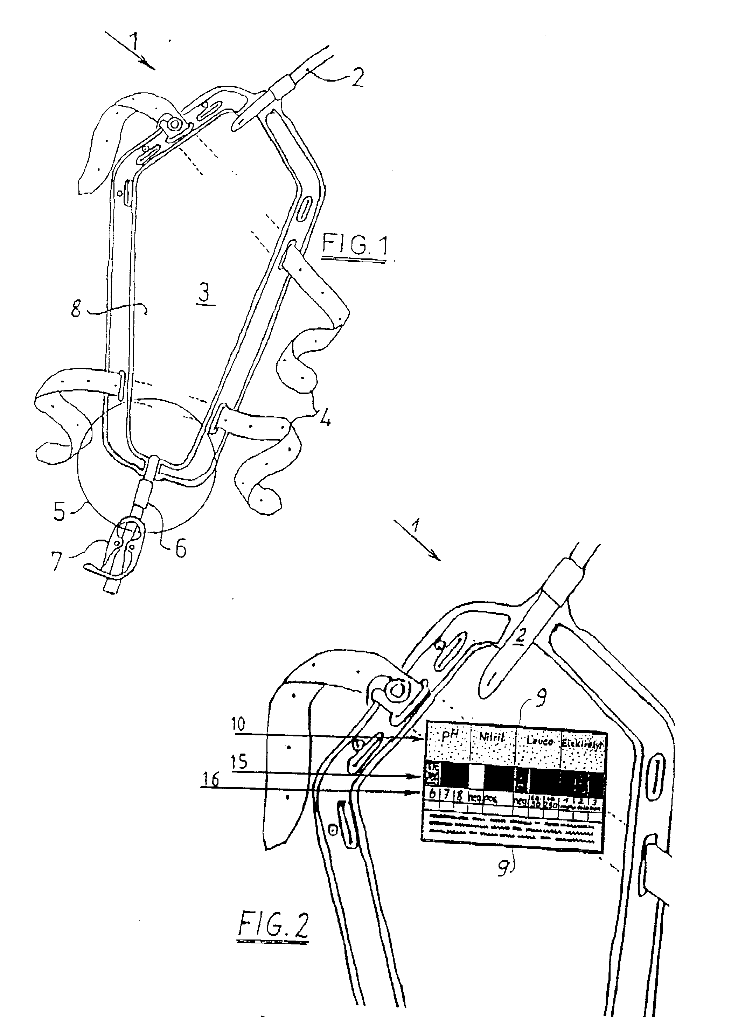

Fig. 1 zeigt eine insgesamt mit 1 bezeichnete Einrichtung zur Aufnahme unkontrollierbar ausgeschiedenen Urins, der über einen mit dem Körper verbundenen Adapter (nicht dargestellt) und durch einen Einleitschlauch 2 in einen Auffangbeutel 3 fließt. Der Auffangbeutel 3 mit zwei Befestigungsriemen 4 wird am Bein getragen und ist an seinem unteren verjüngten Ende 5 mit einem kurzen Austrittsschlauch 6 versehen, der von einer Klammer 7 durch aufhebbare Federkraft zugeklemmt ist. Alle Gegenstände 1 bis 6 sind aus flexiblem durchsichtigen Material.

Fig. 2 zeigt von der Einrichtung 1 gem. Fig. 1 eine Aufsicht auf die Innenwandung 8 des Auffangbeutels 3, die vor Verschweißen mit einer Kontrollkarte 9 beklebt wird. Auf der Kontrollkarte 9 ist oben ein Indikatorenfeld 10 angeordnet, das verschiedene Indikatorenabschnitte 11 bis 14 aufweist, deren Indikatoren auf pH-, Nitrit-, Leukozyt-, und Elektrolytwerte ansprechen. Die Indikatoren sind zunächst weiß; wenn sie später dem Urin exponiert sind, können sie Farben bilden. Im sauren Medium wird sich der Abschnitt 11 des ph-Wertindikators gelb färben und im alkalischen Medium wird er sich blau färben. Auch die übrigen Indikatorenabschnitte 12 bis 14 können verschiedene Farben annehmen, so daß neben der qualitativen Anzeige auf den Abschnitten 11 bis 14 auch eine quantitative Anzeige erfolgt.

Unter dem Indikatorenfeld 10 ist die Kontrollkarte 9 mit einem Farbenfeld 15 bedruckt, das unter jedem Indikatorenabschnitt 11 bis 14 mehrere Farben zeigt, die auch der darüber befindliche Indikator annehmen kann.

In einer dritten Zeile unter dem Farbenfeld 15 erstreckt sich ein Bedeutungsschriftfeld 16. Für den träger der Einrichtung ist von jedem Indikatorabschnitt 11 bis 14 die Quantisierungsaussage maßgebend, über der Farbgleichheit in den beiden oberen Zeilen besteht.

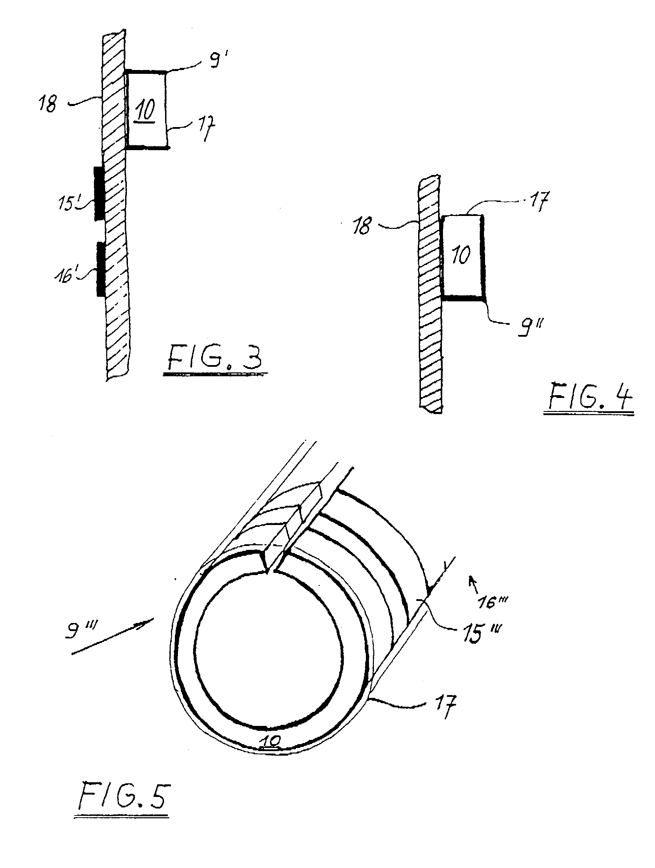

Das Indikatorenfeld 10 ist auf der urinexponierten, der Innenwandung 8 abgewandten Seite mit einer Membran 17 abgedeckt, wie aus Fig. 3 ersichtlich, die eine aus sehr kleinen Löchern gebildete Perforation aufweist. Die Membran 17 läßt den Urin nur langsam zum Indikatorenfeld 10 durchdringen. Auf diesem Wege wird verhindert, daß in den noch leeren Auffangbeutel 3 eintretender Vorlaufurin mit hoher Keimkonzentration eine überkritische Anzeige bewirkt, die vom Mittelstrahlurin normalerweise nicht ausgelöst werden kann. Ebenso kann auch der vom Mittelstrahl abweichende Resturin nicht allein anzeigebestimmend wirken; vielmehr trifft der Urin als Folge des verlangsamten Durchtritts durch die Membran 17 gut gemischt auf das Indikatorenfeld 10.

Die Indikatorsubstanzen des Indikatorenfeldes 10 sind in einem Material gebettet, das bei Feuchtigkeit quillt, so daß beim Aufquellen ein Druck gegen die Membran 17 aufgebraut und der Urindurchgang erschwert wird. Damit wird einem Ausschwemmen der farbbildenden Indikatorsubstanzen entgegengewirkt, und die Anzeigewerte können auch nach vielen Stunden unverfälscht abgelesen werden.

Die in Fig. 3 im Querschnitt dargestellte Kontrollkarte 9′ besteht lediglich aus dem Indikatorenfeld 10, das mit dem der Fig. 2 identisch ist. Farben- und Bedeutungsschriftfeld 15′ und 16′ für die Kontrollkarte 9′ sind an passender Stelle auf den Auffangbeutel 3 gedruckt, vorzugsweise auf dessen Außenwandung 18.

Die in Fig. 4 dargestellte Kontrollkarte 9˝ ist auf der der Innenwandung 8 abgewandten Seite des Indikatorenfeldes 10′ versiegelt. Die für den Urindurchlaß verantwortliche Membran 17′ erstreckt sich über die Schmalseite der Kontrollkarte 9˝. Durch die stark verkleinerte Membranfläche ergibt sich ein entsprechend verlangsamter Urindurchfluß.

Angemerkt sei, daß die perforierte Membran 17 durch eine semipermeable Membran ersetzbar ist.

In Fig. 5 ist eine aufgerollte Kontrollkarte 9˝′ dargestellt, die ansonsten wie die zuvor beschriebenen Kontrollkarten 9,9′ und 9˝ funktioniert. Bei dieser Ausführung ist der Druckanstieg aufgrund des beschriebenen Quellvorgangs besonders stark ausgeprägt.

## Patentansprüche

1. Einrichtung zur Aufnahme unkontrollierbar ausgeschiedenen Urins, der durch einen Einleitschlauch in eine durchsichtige Auffangvorrichtung gelangt, wobei zur Kontrolle des aufgefangenen Urins hinsichtlich sich anbahnender und bestehender Keiminfektionen urinexponierte Indikatoren (10) vorgesehen sind,
**dadurch gekennzeichnet**, daß die urinexponierten Indikatoren (10) auf einer in der Auffangvorrichtung (3) befestigten Kontrollkarte (9,9′,9˝, 9˝′) aufgetragen sind, und daß die Kontrollkarte (9 bis 9˝′) auf ihrer urinexponierten Seite mit einer Membran (17) überzogen ist, die einerseits die Urinaufnahme verlangsamt und andererseits einer Ausschwemmung von Indikatorsubstanzen entgegenwirkt.

2. Einrichtung nach Anspruch 1,
**dadurch gekennzeichnet**,
daß die Membran (17) eine Perforation aus sehr kleinen Löchern aufweist.

3. Einrichtung nach Anspruch 1,
**dadurch gekennzeichnet**,
daß die Membran (17) aus einem semipermeablen Material besteht.

4. Einrichtung nach einem der vorstehenden Ansprüche,
**dadurch gekennzeichnet**,
daß die Auffangvorrichtung (3) neben den Indikatoren ein Vergleichsfarbenfeld (15) trägt.

5. Einrichtung nach Anspruch 4,
**dadurch gekennzeichnet**,
daß dem Vergleichsfarbenfeld (15) ein Bedeutungsschriftfeld (16) zugeordnet ist.

6. Einrichtung nach den Ansprüchen 4 und 5,
**dadurch gekennzeichnet**,
daß das Vergleichsfarben -und das Bedeutungsschriftfeld (15 und 16) auf der Außenseite der Auffangvorrichtung (3) aufgedruckt sind.

7. Einrichtung nach Anspruch 2,
**dadurch gekennzeichnet**,
daß die Kontrollkarte (9˝′) zu einem Zylinder (Fig.5) gerollt ist und daß die Membran (17) den Zylinder (Fig.5) in seiner Form hält.

8. Einrichtung nach Anspruch 2,
**dadurch gekennzeichnet**,
daß die Kontrollkarte (9) Zylinderform hat und daß die unrinexponierte Membran (17) die Innenfläche des Zylinders überdeckt.

9. Einrichtung nach den Ansprüchen 2 und 7,
**dadurch gekennzeichnet**,
daß die Kontrollkarte (9 bis 9˝′) aus einem saugfähigen Material besteht, da nach Feuchtigkeitsaufnahme quillt, wobei der Urinaustausch durch die Löcher behindert wird.

10. Einrichtung nach den Ansprüchen 2 und 7,
**dadurch gekennzeichnet**,
daß die Kontrollkarte (9 bis 9˝′) aus einem Material besteht, das nach Feuchtigkeitsaufnahme kristallisiert.

11. Einrichtung nach den Ansprüchen 2 und 7,
**dadurch gekennzeichnet**,
daß den Indikatoren oder dem Indikatorträger eine Substanz zugegeben ist, die durch physikalische oder chemische Reaktion den Urinaustausch enden läßt.

12. Einrichtung nach einem der vorstehenden Ansprüche,
**dadurch gekennzeichnet**,
daß durch strukturelle Veränderung der Membran (17) der Urinaustausch gesteuert wird.

## Claims

1. Appliance for receiving uncontrollably excreted urine which passes through an introduction tube into a transparent collecting device, wherein urine-exposed indicators (10) are provided to monitor the collected urine with respect to incipient and existent microbial infections, characterised in that the urine-exposed indicators (10) are applied to a monitoring card (9,9′,9˝,9˝′) fixed in the collecting device (3) and in that the monitoring card (9 to 9˝′) is coated on its urine-exposed side with a membrane (17) which, on the one hand, slows down urine uptake and, on the other hand, counteracts extraction of indicator substances.

2. Appliance according to Claim 1, characterised in that the membrane (17) has a perforation comprising very small holes.

3. Appliance according to Claim 1, characterised in that the membrane (17) consists of a semipermeable material.

4. Appliance according to any of the preceding claims, characterised in that the collecting device (3) has a comparison colour field (15) in addition to the indicators.

5. Appliance according to Claim 4, characterised in that a significance entry field (16) is assigned to the comparison colour field (15).

6. Appliance according to Claims 4 and 5, characterised in that the comparison colour field and the significance entry field (15 and 16) are printed on the outside of the collecting device (3).

7. Appliance according to Claim 2, characterised in that the monitoring card (9˝′) is rolled into a cylinder (Fig. 5), and in that the membrane (17) maintains the shape of the cylinder (Fig. 5).

8. Appliance according to Claim 2, characterised in that the monitoring card (9) is cylindrical, and in that the urine-exposed membrane (17) covers the inner surface of the cylinder.

9. Appliance according to Claims 2 or 7, characterised in that the monitoring card (9 to 9˝′) consists of an absorbent material which swells after uptake of moisture, with urine exchange through the holes being obstructed.

10. Appliance according to Claims 2 or 7, characterised in that the monitoring card (9 to 9˝′) consists of a material which crystallises after uptake of moisture.

11. Appliance according to Claims 2 or 7, characterised in that a substance which by physical or chemical reaction terminates urine exchange is added to the indicators or the indicator carrier.

12. Appliance according to any of the preceding claims, characterised in that urine exchange is controlled by structural alteration of the membrane (17).

## Revendications

1. Dispositif pour recueillir l'urine de mictions involontaires, qui arrive dans un dispositif de réception transparent par un tuyau, des indicateurs exposés à l'urine étant prévus pour contrôler l'urine recueillie pour déceler des infections présentes ou susceptibles de se produire, caractérisé en ce que les indicateurs exposés à l'urine (10) sont rapportés sur une carte de contrôle (9, 9′, 9˝, 9˝′) fixée sur le dispositif de réception (3), et en ce que la carte de contrôle (9 à 9˝′) est revêtue de son côté exposé à l'urine d'une membrane (17), qui, d'une part ralentit la réception d'urine et, d'autre part, empêche le lessivage de substances indicatrices.

2. Dispositif selon la revendication 1, caractérisé en ce que la membrane (17) est perforée de très petits trous.

3. Dispositif selon la revendication 1, caractérisé en ce que la membrane (17) est constituée d'un matériau semiperméable.

4. Dispositif selon l'une quelconque des revendications précédentes, caractérisé en ce que le dispositif de réception (3) est muni, à proximité des indicateurs, d'un champ de comparaison de coloration (15).

5. Dispositif selon la revendication 4, caractérisé en ce que le champ de comparaison de coloration (15) est associé à un champ de légende (16).

6. Dispositif selon les revendications 4 et 5, caractérisé en ce que les champs de comparaison de coloration et de légende (15, 16) sont imprimés du côté extérieur du dispositif de réception (3).

7. Dispositif selon la revendication 2, caractérisé en ce que la carte de contrôle (9˝′) est enroulée en cylindre (figure 5) et en ce que la membrane (17) maintient la forme cylindrique.

8. Dispositif selon la revendication 2, caractérisé en ce que la carte de contrôle (9) est en forme de cylindre et en ce que la membrane exposée à l'urine (17) recouvre la face interne du cylindre.

9. Dispositif selon la revendication 2 ou 7, caractérisé en ce que la carte de contrôle (9 à 9˝′) est constituée d'un matériau absorbent qui se dilate après absorption, d'où il résulte que l'échange d'urine par les trous est freiné.

10. Dispositif selon la revendication 2 ou 7, caractérisé en ce que la carte de contrôle (9 à 9˝′) est constituée d'un matériau qui se cristallise après avoir absorbé de l'humidité.

11. Dispositif selon la revendication 2 ou 7, caractérisé en ce qu'il est ajouté une substance aux indicateurs ou au support des indicateurs, qui fait cesser l'échange d'urine par une réaction physique ou chimique.

12. Dispositif selon l'une quelconque des revendications précédentes, caractérisé en ce que l'échange d'urine est déterminé par une modification structurelle de la membrane (17).
